# FASCICULE DE BREVET EUROPEEN

(11) **EP 2 651 511 B1**
(45) Date de publication et mention de la délivrance du brevet: **05.10.2016**
(21) Numéro de dépôt: 11807763.5
(22) Date de dépôt: 12.12.2011
(51) Int. Cl.: A61N 5/06, A61N 5/067

(54) **PROCEDE DE TRAITEMENT NON-THERAPEUTIQUE DE LA PEAU GRASSE NON ACNEIQUE**
NICHT THERAPEUTISCHES VERFAHREN ZUR BEHANDLUNG VON FETTIGER HAUT OHNE AKNE
NON-THERAPEUTIC METHOD FOR TREATING NON-ACNEIC OILY SKIN

(30) Priorité: 13.12.2010 FR 1060447; 27.12.2010 US 201061427237 P
(43) Date de publication de la demande: 23.10.2013
(73) Titulaire: L'Oréal, 75008 Paris (FR)
(72) Inventeur: GRANGER, Corinne, 75006 Paris (DE); PERNOT, Hélène, 75005 Paris (FR)
(74) Mandataire: Nony
(86) Numéro de dépôt international: PCT/IB2011/055611
(87) Numéro de publication internationale: WO 2012/080935

(56) Documents cités:
- WO-A1-2006/012752
- WO-A2-03/001984
- WO-A2-2007/106501
- DE-A1- 19 830 222
- US-A1- 2002 173 833
- US-A1- 2003 032 950
- US-A1- 2003 069 618
- US-A1- 2005 154 382
- US-A1- 2006 212 025
- US-A1- 2007 198 004
- US-A1- 2007 217 199
- US-A1- 2009 012 586

## Description

La présente invention concerne le traitement cosmétique, i.e. non thérapeutique, de la peau grasse non acnéique.

La peau est riche en glandes sébacées, et est à renouvellement continuel. La sécrétion de sébum est un phénomène normal et utile à la peau comme à la chevelure. Le sébum constitue normalement un agent hydratant de l'épiderme. Il est le produit naturel de la glande sébacée qui constitue une annexe de l'unité pilosébacée. Il s'agit essentiellement d'un mélange plus ou moins complexe de lipides. Le sébum protège la peau ainsi que le cuir chevelu et assure la brillance des cheveux en lubrifiant la cuticule.

Malheureusement une hypersécrétion de sébum ou séborrhée, peut entraîner des désordres esthétiques. Ainsi, une sécrétion excessive de sébum peut se traduire par une peau grasse d'aspect brillant ou luisant et elle peut aussi favoriser l'apparition d'un état pelliculaire gras du cuir chevelu ou pellicules grasses. Elle peut s'accompagner d'une augmentation de la taille de pores. Par exemple, le stress, la fatigue, la période hivernale peuvent être des facteurs renforçant ces états chez la majorité des personnes. Parmi la population à peau grasse, on peut trouver des sujets ayant des troubles endocriniens ou neurologiques, ou des sujets obèses. On peut également trouver des adolescents, des personnes souffrant de surplus d'hormones (notamment masculines), des femmes en période d'activité menstruelle ou des femmes ménopausées ayant la peau grasse.

Il existe donc un besoin pour remédier à ces problèmes en proposant un procédé de traitement de la peau grasse.

On connaît par les demandes de brevet US 2006/0212025 et US 2006/0200213 des procédés de traitement de l'acné, visant à réduire la prolifération de *P. Acnes.*

Dans les brevets US 6 183 773 et US 6 600 951 sont présentés des procédés de traitement des dysfonctionnements de la glande sébacée au moyen d'une lumière émise par un laser.

La demande US 2009/0299268 a pour objectif de traiter l'acné. Le traitement permet de réduire la sécrétion de sébum et provoque l'éradication des bactéries.

La demande GB 2 356 570 enseigne de traiter l'acné par l'émission de lumière à trois différentes longueurs d'onde, à savoir comprises dans les plages suivantes : 365-465 nm, 585-645 nm, et 646-710 nm.

Enfin, la demande US 2005/0055070 porte sur le traitement de l'acné par la lumière pour détruire les bactéries, grâce à la stimulation de la production de radicaux libres par réaction photochimique.

Le document US2007/0198004 A1 porte sur le traitement de l'acné et sur les traitements cosmétiques par la lumière.
L'invention est définie dans les revendications.

L'invention vise à traiter la peau grasse non acnéique en proposant un procédé de traitement cosmétique (non thérapeutique) de la peau grasse non acnéique, comportant les étapes consistant à :
- exposer la peau grasse non acnéique à une première lumière quasi-monochromatique d'origine artificielle ayant un pic dominant de longueur d'onde comprise entre 300 et 700 nm, mieux entre 400 et 650 nm, encore mieux entre 560 et 620 nm, notamment de l'ordre d'environ 590 nm.

On entend par lumière monochromatique, une lumière qui n'est constituée que d'une seule longueur d'onde. Selon l'invention de ce procédé, on entend par lumière quasi-monochromatique, une lumière émettant un spectre de longueur d'ondes présentant un pic dominant à une longueur d'onde. Selon l'invention, ce spectre a une largeur spectrale à mi-hauteur d'au plus ±50nm et une largeur spectrale à la base d'au plus ±100nm. On définit la largeur spectrale à mi-hauteur comme la largeur du spectre au niveau de la moitié de la puissance du pic dominant. On définit la largeur spectrale à la base comme la largeur du spectre au niveau de 10% de la puissance du pic dominant.

La Demanderesse a constaté qu'il était possible de traiter ainsi des peaux qui n'ont pas développé de manifestation clinique de l'acné afin de rendre la peau moins grasse, moins luisante et moins brillante.

Par « peau » on désigne la peau du visage, du corps, et le cuir chevelu. La peau traitée peut être ridée ou non ridée.

Par « peau non acnéique », on désigne une peau saine dépourvue des manifestations cliniques de l'acné, telles que la présence de nombreux boutons d'acné. Autrement dit, sur toute la zone de peau exposée à la lumière avec le procédé selon l'invention, la peau n'est pas cliniquement acnéique. La zone entière de peau exposée à la lumière est dépourvue d'une zone ayant développé de l'acné. Par « manifestations cliniques de l'acné », on entend la présence sur la peau de lésions d'acné.

Une peau grasse hyperséborrhéique est caractérisée par une sécrétion et une excrétion exagérée de sébum. Par « peau grasse » on désigne une peau qui obtient un score supérieur à 95 µg/cm² au sébumètre. Par « peau très grasse », on désigne un score au sébumètre supérieur à 120 µg/cm² et par «peau excessivement grasse », on désigne un score au sébumètre supérieur à 140 µg/cm².

Une telle peau est en outre souvent associée à un défaut de desquamation, un teint luisant, un grain de peau épais, des pores dilatés, ou un relief irrégulier, manifestations ressenties comme des imperfections cutanées ou défauts esthétiques. L'apparence et/ou la visibilité des pores est aussi une caractéristique de la peau grasse. La brillance de la peau est aussi liée à la dilatation des pores. La peau grasse se caractérise aussi par une peau luisante, parfois d'aspect huileux, épaisse, aux pores pilosébacés dilatés.

### Sébumètre

Le sébumètre permet de mesurer la production de sébum dans le temps. La quantité de sébum excrétée à la surface de la peau est évaluée à l'aide d'un Sébumètre^{®} SM180 (COURAGE & KHAZAKA).

Il s'agit d'une méthode photométrique. Un ruban de matière synthétique, qui devient transparent au contact des lipides absorbés, est appliqué sur la zone de mesure durant 30 secondes précisément.

Sa transparence augmente alors proportionnellement avec la quantité de sébum du film hydrolipidique avec lequel il est en contact.

Un enregistrement par réflectométrie permet de quantifier l'augmentation de la lumière transmise et ainsi de déterminer la masse totale de lipides excrétés par unité de surface (en µg.cm⁻²).

Une mesure au niveau du front après délipidation soignée avec de l'alcool à 70° est réalisée.

Puis après une demi-heure de temps, une nouvelle mesure est réalisée. On peut ainsi calculer la quantité de sébum excrétée par unité de surface et par unité de temps.

La quantité de sébum peut aussi être évaluée à l'aide d'un Sebutape^{®}.

### Sebutape^{®}

Le Sebutape^{®} permet de mesurer la quantité de sébum produite pendant une période donnée. On utilise un Sebutape^{®} de référence S100 de la société CuDerm Corp. Tx, USA, également disponible auprès de la société Monaderm. Le Sebutape^{®} est appliqué et pressé délicatement sur la peau au niveau des tempes après délipidation soignée avec de l'alcool à 70°, et laissé en place en contact avec la peau pendant une durée de 30 minutes. Le Sebutape^{®} est ensuite retiré, puis mis en contact avec une feuille en plastique transparente. On détermine visuellement le score en utilisant une échelle appropriée de 0 à 5.

Typiquement, une peau grasse selon l'invention présente un score supérieur à 2.

### Dermascore

Le Dermascore permet de visualiser certaines caractéristiques de la peau en fonction de la polarisation de la lumière, à savoir les pores de la peau avec de la lumière polarisée parallèlement et la couleur de la peau ainsi que les hétérogénéités de la peau avec de la lumière polarisée perpendiculairement. Des images sont saisies et une analyse effectuée par comparaison avec les images d'un atlas.

La présente invention diminue la sécrétion du sébum. Une peau grasse est en outre souvent associée à un défaut de desquamation, un teint luisant, un grain de peau épais, une taille de pores augmentée, manifestations ressenties comme des désordres esthétiques auxquels le traitement selon l'invention vise également à remédier.

Le traitement selon l'invention permet avantageusement de prévenir et/ou traiter l'aspect luisant de la peau. Au sens de la présente invention, on entend par "prévenir" le fait de diminuer au moins en partie le risque de manifestation d'un phénomène donné. La diminution partielle implique que le risque demeure mais à un degré moindre qu'avant la mise en oeuvre de l'invention.

Le traitement conforme à l'invention permet avantageusement de prévenir et/ou traiter une peau d'aspect brouillé, terne et/ou inhomogène, cireuse, jaunâtre, voir d'aspect maladif.

Le traitement conforme à l'invention permet avantageusement de prévenir et/ou traiter les désordres esthétiques associés au cuir chevelu gras, tel qu'une hypersécrétion de sébum, ou séborrhée, qui peut favoriser l'apparition d'un état pelliculaire gras du cuir chevelu ou de pellicules grasses.

Le traitement selon l'invention peut s'avérer ainsi tout particulièrement efficace :
- pour prévenir et/ou traiter les peaux grasses,
- pour améliorer le confort des peaux et cuirs chevelus gras,
- pour traiter et/ou prévenir et/ou éviter les désordres esthétiques du cuir chevelu liés à un excès d'excrétions et/ou de sécrétions de sébum,
- pour prévenir et/ou traiter le cuir chevelu gras, et notamment les états pelliculaires gras du cuir chevelu,
- pour rétablir une écoflore équilibrée du cuir chevelu gras.

Une hypothèse avancée pour expliquer l'effet du traitement, sans être lié par cette explication, est que le traitement selon l'invention agit sur la glande sébacée, en diminuant son activité.

Le procédé peut avoir un effet sur les signes physiologiques et cliniques de la peau grasse, notamment la quantité de sébum et la qualité du sébum. Un autre effet du procédé de traitement selon l'invention peut être de réduire la taille des pores. Un autre effet encore peut être de réduire la visibilité des cicatrices d'acné.

Le procédé selon l'invention est plus particulièrement destiné à traiter des individus ayant entre 20 et 60 ans, mieux entre 25 et 50 ans, alors que leur peau ne souffre pas d'acné.

Le procédé selon l'invention peut être mis en oeuvre en exposant à la source de lumière le visage par zone ou dans son ensemble, le cuir chevelu gras par zone ou dans son ensemble, le corps ou une partie du corps ayant une problématique peau grasse.

Le procédé selon l'invention peut être mis en oeuvre en exposant à la source de lumière le visage par zone ou dans son ensemble, le cuir chevelu gras par zone ou dans son ensemble, le corps ou une partie du corps avec un appareil statique en contact ou non avec la zone traitée.

Par « appareil statique », on entend que l'on n'a pas à déplacer un appareil par rapport au visage ou à la tête.

En variante, l'appareil peut être utilisé successivement sur plusieurs zones afin de couvrir une surface de traitement plus large. Sur chaque zone, l'appareil est maintenu immobile.

Le procédé selon l'invention peut être mis en oeuvre avec un support émettant de la lumière selon l'invention, en contact avec la peau du visage, du cuir chevelu, ou du corps. Le support peut être un matériau émettant de la lumière. Le support peut être au moins partiellement en textile, voire entièrement. Le procédé selon l'invention peut être mis en oeuvre par un appareil à commande tactile, un écran rigide ou souple, un miroir, un boîtier, un verre, émettant de la lumière selon l'invention.

Le traitement ne provoque pas de lésions thermiques, la puissance lumineuse étant faible.

Le spectre de la lumière émise peut comporter une première lumière quasi-monochromatique correspondant à la première lumière ci-dessus, seule ou en combinaison avec une ou plusieurs autres lumières quasi-monochromatiques.

Outre l'exposition à la première lumière quasi-monochromatique telle que définie plus haut, on peut exposer la peau grasse non acnéique à une deuxième lumière quasi-monochromatique d'origine artificielle ayant un pic dominant de longueur d'onde comprise entre 700 et 1000 nm, mieux entre 800 et 900 nm, par exemple de l'ordre de 870 nm. Cette deuxième lumière est une lumière rouge ou infrarouge. L'exposition à la lumière rouge ou infrarouge peut être réalisée simultanément ou successivement à l'exposition à la première lumière mentionnée ci-dessus.

La source produisant la lumière rouge ou infrarouge peut être la même que ou être différente de la source produisant la première lumière mentionnée ci-dessus. On peut effectuer le traitement au moyen d'une unique source de lumière configurée pour émettre deux lumières quasi-monochromatiques distinctes. En variante, on peut effectuer le traitement en utilisant deux sources différentes émettant deux lumières quasi-monochromatiques différentes. Les deux sources peuvent être activées simultanément ou successivement.

On peut encore exposer la peau à une troisième lumière quasi-monochromatique, bleue, ayant un pic dominant de longueur d'onde compris par exemple entre 400 et 450 nm, notamment de l'ordre de 410 à 420 nm. Cette troisième lumière additionnelle peut être émise par une source de lumière différente de la ou des sources de lumière émettant la première lumière et éventuellement la deuxième lumière rouge ou infrarouge.

La première lumière peut être dominante par rapport à la ou aux autre(s) lumières. Le pic dominant de longueur d'onde de la première lumière peut avoir une intensité supérieure, à ou aux autre(s) pic(s) dominants de longueur d'onde des autres lumières. La première lumière peut par exemple représenter plus de 50 %, mieux plus de 60 %, encore mieux plus de 70 %, de l'énergie totale de toute la lumière reçue.

La ou les source(s) de lumière générant la lumière à laquelle la peau est exposée peuvent comporter l'un au moins d'une LED, d'une matrice de LEDs, d'une OLED, d'un laser, d'une lampe à incandescence munie d'un filtre dichroïque, cette liste n'étant pas limitative.

L'utilisation d'OLED peut être préférée dans la mesure où elles permettent d'appliquer la lumière au plus prêt de la peau. Elles peuvent être intégrées à un masque ou un patch.

La ou les source(s) de lumière peuvent comporter au moins une LED quasi-monochromatique. Par « LED quasi-monochromatique » on désigne une LED dont le spectre d'émission comporte un pic de longueur d'onde dominante de largeur spectrale à mi-hauteur d'au plus ±50nm et une largeur spectrale à la base du pic d'au plus de ±100nm. En variante, la source de lumière peut comporter au moins une LED bichromatique, c'est-à-dire dont le spectre d'émission comporte plusieurs lumières quasi-monochromatiques, par exemple deux, lumières quasi-monochromatiques, par exemple une lumière quasi-monochromatique dont le pic dominant est entre 560 nm et 620 nm, d'intensité plus importante, et une lumière quasi-monochromatique dont le pic dominant est entre 700 nm et 1000 nm, d'intensité plus faible.

La ou les sources de lumière peuvent être autres qu'un laser.

La lumière, notamment la première lumière de longueur d'onde comprise entre 300 et 700 nm telle que définie plus haut, peut être pulsée. Les éventuelles deuxième et/ou troisième lumières peuvent également être pulsées, par exemple avec des pulses de même durée et des interpulses de même durée que la première lumière. En variante, les caractéristiques de pulsation de ces trois lumières peuvent différer entre elles. Dans une autre variante, l'une au moins des deuxième et troisième lumières peut être continue. Les impulsions peuvent avoir une durée comprise entre 100 et 500 ms, mieux entre 200 et 300 ms, par exemple de l'ordre de 250 ms. La lumière peut être pulsée avec des interpulses d'une durée comprise entre 50 et 200 ms, mieux entre 70 et 150 ms, par exemple de l'ordre de 100 ms.

En variante, toute la lumière émise peut être continue.

La puissance surfacique de la lumière reçue par la peau grasse non acnéique au cours d'un traitement peut être inférieure à 40 mW/cm², de préférence comprise entre 1 et 20 mW/cm², mieux comprise entre 1 et 10 mW/cm², par exemple de l'ordre de 5 mW/cm².

L'énergie surfacique reçue par la peau grasse non acnéique au cours du traitement, sur une journée, peut être inférieure à 4 J/cm², mieux comprise entre 20 mJ/cm² et 1 J/cm², par exemple de l'ordre de 175 mJ/cm².

On peut exposer une même région de peau grasse non acnéique à la lumière conformément à l'invention pendant une durée inférieure à 20 minutes, par exemple comprise entre 20 et 100 secondes, par exemple de l'ordre de 35 secondes ou de 70 secondes.

Dans un exemple de mise en oeuvre de l'invention, lorsque la peau est disposée à une distance comprise entre 0 et 10 cm de la source de lumière, la puissance surfacique reçue est inférieure à 40 mW/cm² et l'énergie surfacique reçue inférieure à 4 J/cm².

On peut effectuer ledit traitement au moins une fois dans la journée au moins un jour par semaine, mieux deux jours par semaine, ou en variante cinq jours par semaine, voire tous les jours, pendant une période d'au moins deux semaines.

Dans un exemple de mise en oeuvre de l'invention, on effectue ledit traitement au moins une fois par semaine pendant une durée comprise entre deux et douze semaines.

La peau ainsi traitée peut être grasse, très grasse, voire excessivement grasse, comme défini plus haut.

Le traitement peut être effectué sur la peau d'un sujet après évaluation de la peau de ce sujet, l'évaluation ayant permis de déterminer la nature « grasse » de la peau du sujet. L'évaluation peut résulter d'une mesure effectuée sur la peau du sujet, par exemple au moyen d'un sébumètre™ ou d'un sébutape™, ou encore par autoévaluation. L'autoévaluation peut par exemple résulter des réponses données par le sujet à un questionnaire détaillé concernant des sensations de peau grasse ou de peau luisante, ou des observations visuelles de l'état de sa peau dans différentes conditions d'observations, par exemple à certains moments de la journée ou un certain temps après la toilette.

Le traitement peut s'effectuer avec une lumière dont la puissance de la longueur d'onde dominante dans la gamme 700 nm-1000 nm est inférieure au moins au quart de la puissance de la longueur d'onde dominante dans la gamme 560 nm-620 nm.

L'invention a encore pour objet, indépendamment ou en combinaison avec ce qui précède, un procédé de traitement cosmétique de la peau grasse non acnéique, comportant l'étape consistant à :
- exposer la peau non acnéique à une source de lumière de longueur d'onde dominante comprise entre 300 et 700 nm, mieux entre 400 et 650 nm, mieux entre 560 et 620 nm, de préférence de l'ordre de 590 nm de façon à réduire la production de sébum de 8 % au moins et à réduire d'au moins 7% le *P. Acnes* par une modification des conditions nutritionnelles du développement de *P. Acnes.*

Le traitement selon l'invention peut être effectué sans application de produit. Plus précisément, on peut ne pas appliquer de produit sur la zone à traiter notamment dans l'heure avant l'exposition à la lumière. On peut ne pas appliquer de produit sur la zone traitée notamment dans l'heure après l'exposition à la lumière.

### Prétraitement

En variante, le traitement peut être mené avec application de produit. Le procédé peut ainsi comporter en outre l'étape suivante :
- appliquer sur la peau, préalablement à l'exposition à la lumière, un produit cosmétique.

### Post-traitement

En variante ou additionnellement, le traitement peut être suivi d'une application de produit. Le procédé peut ainsi comporter en outre l'étape suivante :
- appliquer sur la peau, après l'exposition à la lumière, un produit cosmétique.

### Compositions

Les produits de prétraitement ou de post-traitement peuvent être tels que décrit ci-dessous.

Il peut s'agir par exemple d'un produit nettoyant tel que Cétaphil (eau, Cetyl alcool, Propylène glycol, sodium lauryl sulfate, alcool stearyl, methylparaben, propylparaben, butylparaben). Le produit appliqué peut être relativement peu agressif, permettant seulement de nettoyer légèrement la peau, par exemple pour enlever le maquillage, mais n'étant pas un produit trop dégraissant.

En variante ou additionnellement, il peut s'agir d'un produit hydratant. Le produit hydratant peut par exemple avoir l'une des compositions générales A ou B suivantes :

**Composition A : Crème sous forme d'émulsion eau-dans-huile**

| **Nom chimique** | **Concentration** |
|---|---|
| EAU | QSP 100% |
| GLYCEROL | 7 |
| P-HYDROXYBENZOATE DE METHYLE | 0,3 |
| SULFATE DE MAGNESIUM | 0,7 |
| POLY METHYLCETYL DIMETHYL METHYLSILOXANE OXYETHYLENE (20/75/5 - VISCOSITE : 3000 CST) | 3,75 |
| POLYGLYCERYL-4 ISOSTEARATE | 1,25 |
| ISOHEXADECANE | 7,75 |
| ISONONYL ISONONANOATE | 7,75 |
| P-HYDROXYBENZOATE DE PROPYLE | 0,25 |
| MELANGE DE STEARATE D'ETHYLENE GLYCOL ACETYLE, TRI-STEARATE DE GLYCERYLE | 0,5 |
| POLY ACRYLATE DE STEARYLE | 1,3 |
| ACETATE DE VITAMINE E | 0,2 |
| IODOPROPYNYL BUTYLCARBAMATE | 0,2 |
| ACIDE N-OCTANOYL-5 SALICYLIQUE | 0,3 |
| CYCLOHEXASILOXANE | 8,7 |
| ACRYLATES COPOLYMER | 0,5 |
| PARFUM | 0,2 |
| MICRO-SPHERES DE SILICE AMORPHE | 3 |
| ACIDE GLYCOLIQUE | 0,1 |
| ALGAE EXTRACT | 0,5 |
| ACIDE ETHYLENE DIAMINE TETRACETIQUE, SEL TETRASODIQUE | 0,05 |
| PIDOLATE DE CUIVRE | 0,05 |
| PIDOLATE DE ZINC | 0,25 |
| NYLON-12 (and) CAPRYLOYL SALICYLIC ACID (and) TOCOPHEROL | 2,7 |
| **TOTAL** | **100** |

**COMPOSITION B : Crème sous forme d'émulsion huile-dans-eau**

| **Nom chimique** | **Concentration** |
|---|---|
| EAU | QSP 100 % |
| GLYCEROL | 5 |
| EDTA | 0,2 |
| CONDENSAT D'OXYDE D'ETHYLENE ET D'OXYDE DE PROPYLENE ET D'OXYDE D'ETHYLENE (PM : 14000) (128 OE/54 OP/128 OE) | 0,5 |
| PARTICULES DE NYLON-12 CHARGEES DE TOTAROL D'ACIDE N-OCTANOYL-5-SALICYLIQUE, ET D'ADICE BETA-GLYCYRRHETINIQUE | 0,5 |
| HYDROXYDE DE SODIUM PUR | 0,52 |
| PHENYL TRIMETHYLSILOXY TRISILOXANE (VISCOSITE : 20 CST-PM : 372) | 2 |
| COPOLYMERE DE METHACRYLATE D'ALKYLE GREFFE POLY DIMETHYLSILOXANE | 0,3 |
| COPOLYMERE ACIDE ACRYLIQUE/MATHACRYLATE DE STEARYLE POLYMERISE DANS UN MELANGE ACETATE D'ETHYLE/CYCLOHEXANE | 0,9 |
| FIBRES DE POLYAMIDES 0.9 DTEX DE 0.3 MM DE LONG LAVEES A CHAUD PUIS TAMPONNEES | 2 |
| LACTATE D'ALCOOLS C12-13 RAMIFIES | 2 |
| ACIDE N-OCTANOYL-5 SALICYLIQUE | 0,15 |
| PARFUM | 0,25 |
| COPOLYMERE ACRYLAMIDE/ACRYLAMIDO 2-METHYL PROPANE SULFONATE DE SODIUM EN EMULSION INVERSEE A 40 % DANS ISOPARAFFINE/EAU | 0,5 |
| OXYDE DE TITANE-MICA-OXYDE D'ETAIN (58/41/1) | 0,5 |
| PIDOLATE DE ZINC | 0,25 |
| ACIDE ETHYLENE DIAMINE TETRACETIQUE, SEL TETRASODIQUE | 0,05 |
| ZXTRAIT D'ECORCE D'EPERUA FALCATA | 0,1 |
| ALCOOL ETHYLIQUE | 9 |
| ACIDE CALICYLIQUE EN POUDRE | 0,5 |
| BILLES DE POLYETHYLENE (TAILLE : 8-10 MICRONS) | 3 |
| POUDRE DE POLYETHYLENE | 5 |
| COLORANT | QS |
| **TOTAL** | **100** |

On peut également appliquer une crème topique, comportant un actif. L'activité de l'actif peut être renforcée grâce à l'exposition à la lumière.

On peut également appliquer un produit matifiant, c'est-à-dire un produit ayant un effet immédiat pour remédier au problème de peau grasse, l'exposition à la lumière permettant d'avoir un effet à plus long terme.

On peut encore effectuer une routine de soin, comportant les compositions A et B appliquées successivement. La composition A est par exemple appliquée le soir et la composition B le matin.

Un procédé selon l'invention peut comprendre l'application topique d'une composition sur la peau grasse non acnéique, la peau du visage, et/ou le cuir chevelu.

La composition peut comprendre, dans un milieu physiologiquement acceptable, un actif pour le soin des peaux grasses, par exemple un agent desquamant, par exemple l'acide n-octanoyl-5-salicylique.

La composition peut comprendre, dans un milieu physiologiquement acceptable un ou plusieurs agents de la liste suivante : agent matifiant, agent exfoliant ou charge abrasive, agent sébo-régulateur, agent desquamant, agent apaisant, agent anti-irritant, agent anti-inflammatoire, agent anti-oxydant, agent cicatrisant, agent astringent,

Par « agent matifiant », on entend des agents destinés à rendre la peau visiblement plus mate, moins brillante.

L'effet matifiant de l'agent et/ou de la composition le contenant peut notamment être évalué à l'aide d'un gonioréflectomètre, en mesurant le rapport R entre la réflexion spéculaire et la réflexion diffuse. Une valeur de R inférieure ou égale à 2 traduit généralement un effet matifiant.

L'agent matifiant pourra notamment être choisi parmi un amidon de riz ou un amidon de maïs, la kaolinite, les silices, le talc, un extrait de graines de potiron, des microbilles de cellulose, des fibres végétales, des fibres synthétiques, en particulier de polyamides, des microsphères de copolymères acryliques expansées, des poudres de polyamides, les poudres de silice, les poudres de polytétrafluoroéthylène, les poudres de résine de silicone, les poudres de copolymères acryliques, les poudres de cire, les poudres de polyéthylène, les poudres d'organopolysiloxane réticulé élastomère enrobées de résine de silicone, les poudres composites de talc/dioxyde de titane/alumine/silice, les poudres de silicates mixtes amorphes, les poudres de polymères acryliques, les particules de silicate et notamment de silicate mixte, et leurs mélanges.

Comme exemples d'agents matifiants, on peut citer notamment :
- l'amidon de riz ou de maïs, en particulier un aluminium starch octenyl succinate commercialisé sous la dénomination Dry Flo^{®} par la société National Starch,
- la kaolinite ;
- les silices ;
- le talc ;
- un extrait de graines de potiron tel que commercialisé sous la dénomination Curbilene^{®} par la société Indena ;
- des microbilles de cellulose telles que décrites dans la demande de brevet EP 1 562 562 ;
- des fibres, telles que des fibres de soie, de coton, de laine, de lin, de cellulose extraites notamment du bois, des légumes ou des algues, de polyamide (Nylon^{®}), de cellulose modifiée, de poly-p-phénylène téréphtamide, en acrylique, de polyoléfine, de verre, de silice, d'aramide, de carbone, de Téflon^{®}, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges décrites dans la demande de brevet EP 1 151 742 ;
- des microsphères de copolymères acryliques expansées telles que celles commercialisées par la société EXPANCEL sous les dénominations EXPANCEL 551^{®},
- des charges à effet optique telles que décrites dans la demande de brevet FR 2 869 796, en particulier :
   - les poudres de polyamides (Nylon^{®}), comme par exemple les particules de Nylon 12 du type Orgasol d'Atofina de taille moyenne 10 microns et d'indice de réfraction 1,54,
   - les poudres de silice, comme par exemple les Silica beads SB150 de Miyoshi de taille moyenne 5 microns et d'indice de réfraction 1,45,
   - les poudres de polytétrafluoroéthylène, comme les PTFE ceridust 9205F de Clariant de taille moyenne 8 microns et d'indice de réfraction 1,36,
   - les poudres de résine de silicone comme les Silicon resin Tospearl 145A de GE Silicone de taille moyenne 4,5 microns et d'indice de réfraction 1,41,
   - les poudres de copolymères acryliques, notamment de poly(méth)acrylate de méthyle, comme les particules PMMA Jurymer MBI de Nihon Junyoki de taille moyenne 8 microns et d'indice de réfraction 1,49, ou les particules Micropearl M100^{®} et F 80 ED^{®} de la société Matsumoto Yushi-Seiyaku ;
   - les poudres de cire comme les particules Paraffin wax microease 114S de micropowders de taille moyenne 7 microns et d'indice de réfraction 1,54,
   - les poudres de polyéthylène, notamment comprenant au moins un copolymère éthylène/acide acrylique, et en particulier constituées de copolymères éthylène/acide acrylique comme les particules Flobeads EA 209 de Sumitomo (de taille moyenne 10 microns et d'indice de réfraction 1,48),
   - les poudres d'organopolysiloxane réticulé élastomère enrobées de résine de silicone, notamment de résine silsesquioxane, comme décrit par exemple dans le brevet US 5 538 793. De telles poudres d'élastomère sont vendues sous les dénominations « KSP-100 », « KSP-101 », « KSP-102 », « KSP-103 », « KSP-104 », « KSP-105 » par la société SHIN ETSU, et
   - les poudres composites de talc/dioxyde de titane/alumine/silice comme celles vendues sous la dénomination Coverleaf AR-80 par la société Catalyst & chemicals,
   et leurs mélanges ;
- des composés absorbant et/ou adsorbant le sébum tels que décrit dans la même demande de brevet FR 2 869 796. On peut citer notamment :
   - les poudres de silice, comme par exemple les microsphères de silice poreuses vendues sous la dénomination "SILICA BEADS SB-700" commercialisées par la société MYOSHI, les "SUNSPHERE^{®} H51", "SUNSPHERE^{®} H33" , "SUNSPHERE^{®} H53" commercialisées par la société ASAHI GLASS ; les microsphères de silice amorphe enrobées de polydiméthylsiloxane vendues sous la dénomination "SA SUNSPHERE^{®} H-33" et "SA SUNSPHERE^{®} H-53" commercialisées par la société ASAHI GLASS ;
   - les poudres de silicates mixtes amorphes, notamment d'aluminium et de magnésium, comme par exemple celle commercialisée sous la dénomination « NEUSILIN UFL2 » par la société Sumitomo.
   - les poudres de polyamides (nylon^{®}), comme par exemple "l'ORGASOL^{®} 4000" commercialisé par la société ATOCHEM, et
   - les poudres de polymères acryliques, notamment de polyméthacrylate de méthyle, comme par exemple le "COVABEAD^{®} LH85" commercialisé par la société WACKHERR ; de polyméthacrylate de méthyle/diméthacrylate d'éthylène glycol, comme par exemple le "DOW CORNING 5640 MICROSPONGE^{®} SKIN OIL ADSORBER" commercialisé par la société DOW CORNING, ou le "GANZPEARL^{®} GMP-0820" commercialisé par la société GANZ CHEMICAL ; de polyméthacrylate d'allyle/diméthacrylate d'éthylène glycol, comme par exemple le "POLY-PORE^{®} L200" ou le "POLY-PORE^{®} E200" commercialisés par la société AMCOL ; de copolymère diméthacrylate d'éthylène glycol/méthacrylate de lauryle, comme par exemple le "POLYTRAP^{®} 6603" commercialisé de la société DOW CORNING ;
   - les particules de silicate, telle que la silicate d'alumine ;
   - les particules de silicates mixtes, telles que :
      o les particules de silicate d'aluminium et de magnésium, telles que la saponite ou silicate de magnésium et d'aluminium hydraté avec un sulfate de sodium commercialisée sous la dénomination commerciale Sumecton^{®} par la société Kunimine ;
      o le complexe silicate de magnésium, hydroxyéthylcellulose, huile de cumin noir, huile de courge et phospholipides ou Matipure^{®} de Lucas Meyer.
      et leurs mélanges.

Comme agents matifiants préférés, on pourra utiliser selon l'invention un extrait de graines de potiron, un amidon de riz ou de mais, la kaolinite, des silices, le talc, les poudres de polyamides, les poudres de polyéthylènes, les poudres de copolymères acryliques, les microsphères de copolymères acryliques expansées, les microbilles de résines de silicones, les particules de silicate mixte et leurs mélanges.

### Charges abrasives ou agents exfoliants

Comme agents exfoliants utilisables dans des compositions selon l'invention, on peut citer par exemple des particules exfoliantes ou gommantes d'origine minérale, végétale ou organique. Ainsi, on peut utiliser par exemple des billes ou de la poudre de polyéthylène, de la poudre de nylon, de la poudre de polychlorure de vinyle, de la pierre ponce, des broyats de noyaux d'abricots ou de coques de noix, de la sciure de bois, des billes de verre, l'alumine, et leurs mélanges.

On peut citer aussi l'Exfogreen^{®} de Solabia (extrait de bambou), des extraits d'akenes de fraises (Akenes de fraise de Greentech), de la poudre de noyau de pêche, la poudre de noyau d'abricot, et enfin dans le domaine des poudres végétales à effet abrasif, citons la poudre de noyaux d'airelles (cranberry).

Comme charges abrasives ou agents exfoliants préférés selon l'invention, on citera la poudre de noyaux de pêche, la poudre de noyaux d'abricot, la poudre de noyaux d'airelles, les extraits d'akènes de fraise, les extraits de bambou.

### Agents sébo-régulateurs ou anti-séborrhéiques

Par agents "séborégulateurs ou anti-séborrhéiques ", on entend notamment des agents capables de réguler l'activité des glandes sébacées.

On peut citer notamment :
- l'acide rétinoïque, le peroxyde de benzoyle, le soufre, la vitamine B6 (ou pyridoxine), le chlorure de sélénium, la criste marine ;
- les mélanges d'extrait de canelle, de thé et d'octanoylglycine tel que le Sepicontrol A5 TEA de chez Seppic ;
- le mélange de capryloyl glycine, de sarcosine et d'extrait de cinnamomum zeylanicum commercialisé notamment par la société SEPPIC sous la dénomination commerciale Sepicontrol A5^{®} ;
- les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc ;
- les sels de cuivre, en particulier le pidolate de cuivre ;
- des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par la société MARUZEN ;
- les extraits de reine des prés (spiraea ulamaria) tel que celui vendu sous la dénomination Sébonormine^{®} par la société Silab ;
- les extraits d'algue laminaria saccharina tel que celui vendu sous la dénomination Phlorogine^{®} par la société Biotechmarine ;
- les mélanges d'extraits de racines de pimprenelle (sanguisorba officinalis/poterium officinale), de rhizomes de gingembre (zingiber officinalis) et d'écorce de cannelier (cinnamomum cassia) tel que celui vendu sous la dénomination Sebustop^{®} par la société Solabia ;
- les extraits de graines de lin tel que celui vendu sous la dénomination Linumine^{®} par la société Lucas Meyer ;
- les extraits de Phellodendron tels que ceux vendu sous la dénomination Phellodendron extract BG par la société Maruzen ou Oubaku liquid B par la société Ichimaru Pharcos ;
- les mélanges d'huile d'argan, d'extrait de serenoa serrulata (saw palmetto) et d'extrait de graines de sésame tel que celui vendu sous la dénomination Regu SEB^{®} par la société Pentapharm ;
- les mélanges d'extraits d'epilobe, de terminalia chebula, de capucine et de zinc biodisponible (microalgues) tel que celui vendu sous la denomination Seborilys^{®} par la société green tech ;
- les extraits de Pygeum afrianum tel que celui vendu sous la dénomination Pygeum afrianum sterolic lipid extract par la société Euromed ;
- les extraits de serenoa serrulata tels que ceux vendus sous les dénominations Viapure Sabal par la société Actives International, ou ceux vendus par la société Euromed ;
- les mélanges d'extraits de plantain, de berberis aquifolium et de salicylate de sodium tels que celui vendu sous la dénomination Seboclear^{®} par la société Rahn ;
- l'extrait de clou de girofle tel que celui vendu sous la dénomination Clove extract Powder par la société Maruzen ;
- l'huile d'argan telle que celle vendue sous la dénomination Lipofructyl^{®} par les Laboratoires Sérobiologiques ;
- les filtrats de protéine lactique tels que celui vendu sous la dénomination Normaseb^{®} par la société Sederma ;
- les extraits d'algue laminaria, tel que celui vendu sous la dénomination Laminarghane^{®} par la société Biotechmarine ;
- les extraits de sucre de canne, tel que celui commercialisé sous la dénomination Policasonol^{®} par la société Sabinsa ;
- les oligosaccharides d'algue laminaria digitata tel que celui vendu sous la dénomination Phycosaccharide AC par la société Codif ;
- l'huile de schiste sulfonée, telle que celle vendue sous la dénomination Ichtyol Pale par la société Ichthyol ;
- les extraits d'ulmaire (spiraea ulmaria) tels que celui vendu sous la dénomination Cytobiol Ulmaire par la société Libiol ;
- l'acide sébacique, notamment vendu sous la forme d'un gel de polyacrylate de sodium sous la dénomination Sebosoft par la société Sederma ;
- les glucomannanes extraits de tubercule de konjac et modifié par des chaînes alkylsulfonates tel que celui vendu sous la dénomination Biopol Beta par la société Arch Chemical ;
- les extraits de Sophora angustifolia, tels que ceux vendus sous la dénomination Sophora powder ou Sophora extract par la société Bioland ;
- les extraits de cinchona succirubra bark tel que celui vendu sous la dénomination le Red bark H S par la société Alban Muller ;
- les extraits de quillaja saponaria tel que celui vendu sous la dénomination Panama wood HS par la société Alban Muller ;
- la glycine greffée sur chaîne undécylénique , telle que celle vendue sous la dénomination Lipacide UG OR par la société Seppic ;
- le mélange d'acide oléanolique et d'acide nordihydroguaïarétique , tel que celui vendus sous la forme d'un gel sous la dénomination AC.Net par la société Sederma ;
- l'acide phthalimidoperoxyhexanoïque ;
- le citrate de trialkyle(C₁₂-C₁₃) vendu sous la dénomination COSMACOL^{®} ECI par la société Sasol ; le citrate de trialkyle(C₁₄-C₁₅) vendu sous la dénomination COSMACOL^{®} ECL par la société Sasol ;
- l'acide 10-hydroxydécanoïque, et notamment les mélanges d'acide 10-hydroxydécanoïque, d'acide sébacique et de 1,10-décandiol tels que celui vendu sous la dénomination Acnacidol^{®} BG par la société Vincience ;
- les activateurs PPAR-γ spécifiques comme ceux décrits dans la demande WO 2005/053632 ;
- des extraits de plantes du genre Silybum ;
- des sapogénines ou extraits végétaux en contenant, en particulier les extraits de Dioscorées riches en diosgénine ; et
- des extraits d'Eugenia caryophyllata contenant de l'eugénol et du glucoside d'eugényle,
- et leurs mélanges.

Comme agents séborégulateurs préférés utilisables selon l'invention, on citera :
- la criste marine ;
- les mélanges d'extrait de canelle, de thé et d'octanoylglycine tel que le Sepicontrol A5 TEA de chez Seppic ;
- le mélange de capryloyl glycine, de sarcosine et d'extrait de cinnamomum zeylanicum commercialisé notamment par la société SEPPIC sous la dénomination commerciale Sepicontrol A5^{®} ;
- les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc ;
- les sels de cuivre, en particulier le pidolate de cuivre ;
- les extraits de reine des prés (spiraea ulamaria) tel que celui vendu sous la dénomination Sébonormine^{®} par la société Silab ;
- les extraits d'algue laminaria saccharina tel que celui vendu sous la dénomination Phlorogine^{®} par la société Biotechmarine ;
- les mélanges d'extraits de racines de pimprenelle (sanguisorba officinalis/poterium officinale), de rhizomes de gingembre (zingiber officinalis) et d'écorce de cannelier (cinnamomum cassia) tel que celui vendu sous la dénomination Sebustop^{®} par la société Solabia ;
- les sapogénines ou extraits végétaux en contenant, en particulier les extraits de Dioscorées riches en diosgénine,
- et leurs mélanges.

On peut encore citer les antitranspirants, tels que : les sels d'aluminium et/ou de zirconium ; les complexes d'hydroxychlorure de zirconium et d'hydroxychlorure d'aluminium avec un acide aminé tels que ceux décrits dans le brevet US-3792068 communément connus sous l'appellation "ZAG complexes". De tels complexes sont généralement connus sous l'appellation ZAG (lorsque l'acide aminé est la Glycine). Les complexes ZAG présentent d'ordinaire un quotient Al/Zr allant d'environ 1,67 à 12,5 et un quotient Métal/Cl allant d'environ 0,73 à 1,93. Parmi ces produits on peut citer l'aluminium zirconium octachlorohydrex GLY, l'aluminium zirconium pentachlorohydrex GLY, l'aluminium zirconium tetrachlorohydrate GLY et l'aluminium zirconium trichlorohydrate-GLY.

Parmi les sels d'aluminium, on peut citer le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, les sels d'alun, l'aluminium sulfate, l'aluminium zirconium octachlorohydrate, l'aluminium zirconium pentachlorohydrate, l'aluminium zirconium tetrachlorohydrate, l'aluminium zirconium trichlorohydrate et plus particulièrement l'aluminium chlorhydrate commercialisé par la société REHEIS sous la dénomination MICRODRY ALUMINUM CHLOROHYDRATE ou par la société GUILINI CHEMIE sous la dénomination ALOXICOLL PF 40. Des sels d'aluminium et de zirconium sont par exemple celui commercialisé par la société REHEIS sous la dénomination REACH AZP-908-SUF , des sel d'aluminium « activés » par exemple celui commercialisé par la société REHEIS sous la dénomination REACH 103 ou par la société WESTWOOD sous la dénomination WESTCHLOR 200.

Parmi les autres actifs déodorants, on peut citer également les sels de zinc comme le salicylate de zinc, le sulfate de zinc, le chlorure de zinc, le lactate de zinc, le phénolsulfonate de zinc ; la chlorhexidine et les sels; le monocaprate de diglycérol, le monolaurate de diglycérol , monolaurate de glycérol ; les sels de polyhexaméthylène biguanide.

### Agents anti-microbiens

Par agents anti-microbiens, on entend des agents ayant des effets sur la flore spécifique des peaux grasses, tels que par exemple le *P acnes.*

Ces effets peuvent être soit bactéricides, soit anti-adhésion bactérienne (prévient et/ou réduit l'adhésion des micro-organismes) soit agissant sur le biofilm des bactéries pour éviter leur multiplication.

On peut citer notamment les actifs et conservateurs à activité anti-microbienne cités dans la demande DE10324567, incorporée dans la présente invention par référence.

On peut citer également : un extrait de cône de houblon (HOP CO2-TO extract de Flavex), un extrait de Millepertius (ST John's Wort CO2-TO extract de Flavex), l'acide asiatique, les extraits de racines de scutellaria baicolensis comme dans le BMB - CF de Naturogin, la piroctone olamine, l'acide citrollique, l'acide spérillique, l'ethylhexylglycerine (le Sensiva De Shulke), le caprylate/caprate de gluceryl (Capmul de ABITEC), le calcium sodium phosphosilicate comme Bioactive glasspowder de Schott, les Actysse premier BG de Schott, les oxydes de silicium de Ciba, les Métashines (les dérivés d'argent), les extraits de busserole comme la Gatuline equalizing de Gattefossé, l'acide 10-hydroxy-2 decanoique comme l'Acnacidol P de Vincience, l'ursolate de sodium, l'acide azélaique, le di-iodo-methyl P tolylsulfone ou l'Amical Flowable de Angus, la Malachite de Maprecos, le Zincare de Elementis GMBH, l'arlatone dioic de Unichema, l'acide phthalimidoperoxyhexanoique ou Eureco HC de Chemron Corporation ; l'acide ellagique ; le 2,4,4'-trichloro-2'-hydroxy diphényl éther (ou triclosan), la 1-(3',4'-dichlorophenyl)-3-(4'-chlorophenyl)urée (ou triclocarban), le 3,4,4'-trichlorocarbanilide, le 3',4',5'-trichlorosalicylanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopirox, le ciclopiroxolamine, l'acide undécylenique et ses sels, le peroxyde de benzoyle, l'acide 3-hydroxy benzoïque, l'acide 4-hydroxy benzoïque, l'acide phytique, la N-acétyl-L-cystéine, l'acide lipoïque, l'acide azélaïque et ses sels, l'acide arachidonique, le résorcinol, le 3,4,4'-trichlorocarbanalide, l'octopirox ou piroctone olamine, l'octoxyglycérine ou octoglycérine, l'octanoylglycine (Lipacid C8G^{®} de Seppic), le caprylyl glycol, l'acide 10-hydroxy-2-décanoïque, le dichlorophenyl imidazol dioxolan et ses dérivés décrits dans la demande de brevet WO9318743, les dérivés de zinc et en particulier le pidolate de zinc (Zincidone^{®} de Solabia), les dérivés de cuivre et en particulier le pidolate de cuivre (Cuivridone^{®} de Solabia), l'acide salicylique et ses dérivés, l'iodopropynyl butylcarbamate, le 3,7,11-triméthyldodéca-2,5,10-triénol ou farnesol, les phytosphingosines ; le Sepicontrol^{®} de Seppic, un extrait d'arganier comme l'Argapure LS9710^{®}, le Sebosoft^{®} de Sederma, les sels d'ammonium quaternaires comme les sels de cetyltrimethylammonium, les sels de cétylpyridinium, l'éthanol...et leurs mélanges.

Comme agents prévenant et/ou réduisant l'adhésion des micro-organismes, on peut citer notamment : le phytanetriol et ses dérivés tels que décrits dans la demande de brevet EP 1 529 523, les huiles végétales telles de l'huile de germe de blé, l'huile de calendula, l'huile de ricin, l'huile d'olive, l'huile d'avocat, l'huile d'amande douce, l'huile d'arachide, l'huile de jojoba, l'huile de sésame, l'huile d'amande d'abricot, l'huile de tournesol, l'huile de macadamia, décrites dans le brevet EP 1 133 979, ou encore d'autres corps gras tels que le cocoamphodiacétate disodique, le cocoate de glycéryle oxyéthyléné (7 OE), les Poloxamers, l'hexadécénylsuccinate 18, le palmitate d'octoxyglycéryle, le béhénate d'octoxyglycéryle, l'adipate de dioctyle , le PPG-15 stéaryl éther, le tartrate de dialcools C12-C13 ramifiés décrits dans le brevet EP 1 129 694.

En particulier vis-à-vis de la propagation du *P acnes,* on peut citer le pentylène glycol, le nylon-66 (fibres de polyamides 66), l'huile de son de riz , le Celvol 540 PV alcohol (Polyvinyl alcohol 72962), l'Akorex L de Karlshamns, les dérivés de fructose.

On peut également citer certains tensioactifs ayant un effet antimicrobien comme le cocoampho acétate de sodium ou diacetate disodium tel que le Miranol C2M CONC NP, les bétaines comme la cocoyl bétaine Genagen KB de Clariant, le lauryl ether sulfate de sodium comme l'Emal 270 D de Kao, le décyl glucoside comme le Plantacare 2000 UP, les malate de dialcools C12-13 ramifiés comme le Cosmacol EMI, les monoesters de propylène glycol comme monolaurate, monocaprylate, monocaprate de propylène glycol, le sodium lauroyl oat amino acid comme le Proteol OAT, le lauryl dimethylamine betaine comme le Empigen BB/LS ainsi que les polyammonium quaternaires comme le Quaternium-24 ou Bardac 2050 de Lonza et ceux décrits dans le brevet L'OREAL FR 0 108 283.

Comme agents antimicrobiens préférés, on utilisera dans les compositions de l'invention un agent choisi parmi le caprylyl glycol, les dérivés de zinc dont le pidolate de zinc (Zincidone^{®} de Solabia), les dérivés de cuivre dont le pidolate de cuivre (Cuivridone^{®} de Solabia), l'octoglycérine ou octoxyglycérine, l'acide 10-hydroxy-2-décanoïque, et leurs mélanges.

### Agents desquamants

Par "agent desquamant", on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique et ses dérivés ; les oligofucoses ; l'acide cinnamique ; l'extrait de Saphora japonica ; le resvératrol et certains dérivés d'acide jasmonique ;
- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

Comme autres agents desquamants utilisables dans la composition selon l'invention, on peut citer les oligofructoses, l'EDTA et ses dérivés, les laminaria extract, le o-linoleyl-6D-glucose, le (3-hydroxy-2pentylcyclopentyl)acetic acid, le trilactate de glycérol, l'O-octanyl-6'-D-maltose, la S carboxyméthyl cystéine, les dérivés siliciés de salicylate comme dans le brevet EP 0 796 861, les oligofucase comme dans le brevet EP 0 218 200, les sels d'acide 5-acyl salicylique, des actifs ayant des effets sur la transglutaminase comme dans le brevet EP 0 899 330, l'acide jasmonique et dérivés comme dans les demandes de brevet EP 1 333 022 et EP 1 333 021.

### Exfolactive^{®} de Silab (extrait de fleur de ficus opuntia indica), le Soypon O^{®} de Kawaken fine chemicals (sodium cocoyl sarcosinate)

Comme agents desquamants préférés, on pourra citer les beta-hydroxyacides, tel que l'acide n-octanoyl 5-salicylique ; l'urée ; les acides glycolique, citrique, lactique, tartrique, malique ou mandélique ; l'acide (N-2 hydroxyéthylpiperazine-N-2-éthane) sulfonique (HEPES) ; l'extrait de Saphora japonica ; le miel ; le N-acétyl glucosamine ; le méthyl glycine diacétate de sodium, et leurs mélanges.

Encore plus préférentiellement on utilisera dans les compositions de l'invention un agent desquamant choisi parmi l'acide n-octanoyl 5-salicylique ; l'urée ; l'acide (N-2 hydroxyéthylpiperazine-N-2-éthane) sulfonique (HEPES) ; l'extrait de Saphora japonica ; le miel ; le N-acétyl glucosamine ; le méthyl glycine diacétate de sodium, et leurs mélanges.

### Agents apaisants ou anti-irritants

On peut citer notamment les agents apaisants ou anti-irritants cités dans les demandes WO2004/105736 et DE10324567, incorporées dans la présente invention par référence.

Comme agents apaisants particuliers utilisables dans la composition selon l'invention, on peut citer : les oligomères procyannidoliques, les vitamines E, C B5, B3, le sulfate de dextran, la caféine et ses dérivés, les triterpènes pentacycliques et les extraits de plantes les contenant, l'acide b-glycyrrhétinique et ses sels ou dérivés (le stearyl glycyrrhetate, l'acide 3-stéaroyloxy glycyrrhétique, l'acide glycyrrhétinique monoglucuronide) ainsi que les plantes en contenant (ex : Glycyrrhiza glabra), l'acide oléanolique et ses sels, l'acide ursolique et ses sels, l'acide boswellique et ses sels, l'acide bétulinique et ses sels, un extrait de Paeonia suffruticosa et/ou lactiflora, le salycilate de zinc, les phycosshacharides de la société Codif, un extrait de Laminaria saccharina, les extraits de Centella asiatica, l'huile de Canola, le bisabolol, le diesterphosphorique de vitamine E et C comme le Sepivital EPC^{®} de Seppic, les extraits de camomille, l'allantoïne, les huiles insaturées en oméga 3 telles que les huiles de rosier musca, de cassis, d'Ecchium, de poisson, l'huile de calophilum, des extraits de plancton, la capryloyl glycine, le Seppicalm VG^{®} (nymphea alba et sodium palmitoylproline) de Seppic, un extrait de Pygeum, un extrait de Boswellia serrata, un extrait de Centipeda cunnighami, un extrait d'Helianthus annus en particulier l'Hélioxine de Silab, un extrait de Linum usitatissimum comme la Sensiline de Silab, les tocotriénols, les extraits de Cola nitida, le piperonal, un extrait de clou de girofle, un extrait d'Epilobium angustifolium, l'aloe vera, un extrait de Bacopa moniera, les phytostérols, l'eau de bleuet, l'eau de rose, le dextran comme dans Modulène^{®} de Vincience, un extrait de menthe, en particulier de feuilles de menthe comme le Calmiskin^{®} de Silab, les dérivés d'anis, les bactéries filamenteuse comme Vitreoscilla filiformis tel que décrit dans le brevet EP 761 204, un extrait de rose comme l'Herbasol rose extract, le Stimu-tex AS de Pentapharm, les sels alcalino-terreux notamment le strontium, la niacinamide, et leurs mélanges.

Comme agents apaisants préférés, on utilisera un agent choisi parmi un extrait de rose, un extrait de clou de girofle, le dextran comme dans Modulène^{®} de Vincience, un extrait de menthe comme le Calmiskin^{®} de Silab, un mélange d'un extrait de nymphea alba et sodium palmitoylproline comme le Seppicalm VG^{®} de Seppic, des dérivés d'anis, un extrait de Paeonia suffruticosa et/ou lactiflora, et leurs mélanges.

### Agents anti-inflammatoires

On peut citer notamment les agents anti-inflammatoires cités dans les demandes WO2004/105736 et DE10324567, incorporées dans la présente invention par référence.

Comme agents anti-inflammatoires particuliers utilisables selon l'invention, on peut citer la cortisone, l'hydrocortisone, l'indométhacine, la bétaméthasone, l'acide azéalique, l'acétominophène, le diclofénac, le propionate de clobetasol et leurs mélanges.

Comme agent anti-inflammatoire préféré, on citera l'acide azélaique.

### Agents antioxydants

On entend les agents ayant une activité anti-oxydante (qui empêchent l'oxydation du squalène et la formation des comédons).

On peut citer notamment le tocophérol et ses esters, en particulier l'acétate de tocophérol ; le BHT et le BHA.

On peut également citer les polyphénols, l'acide tannique, l'époigallocathéchines et les extraits naturels en contenant, les anthocyanes, les extraits de romarin, les extraits de feuilles d'olivier, le thé vert, le resvératrol et ses dérivés, le Pycnogénol, l'ergothinéine, la N acétylcystéine, la biotine, les chélatants, l'idébénone, des extraits végétaux come le Pronalen Bioprotect TM de la société Provital, les antiradicalaires comme la vitamine E, le co enzyme Q10, les bioflavonoides, les SOD, le phytantriol, les lignanes, la mélatonine, les pidolates, le gluthation.

### Argents cicatrisants

Comme exemples d'agents cicatrisants, on peut citer notamment :
l'allantoine, l'urée, l'huile de germe de blé, certains acides aminés comme l'hydroxyproline, l'arginine, la sérine, et aussi des extraits de lys blanc (ex : le Phytélène Lys 37EG 16295 de Indena), un extrait de levures comme le cicatrisant LS 7225B de LS (Cognis), l'huile de tamanu, l'extrait de saccharomyces cerevisiae ou Biodynes TRF de Arch Chemical, les extraits d'avoine, le chitosane et dérivés, les extraits de carotte, l'extrait d'artemia ou GP4G de Vincience, l'acexamate de sodium, des extraits de lavandin, des extraits de miel ou de propolis, l'acide ximeninique et ses sels tel que acido ximeninico de Indena, l'huile de rosa rugosa, le Souci Ami Liposolible d'Alban Muller, des extraits de prêle, l'herbasol citron de Cosmetochem, des extraits d'helichryse, des béta-glucan et dérivés, du beurre de karité et ses fractions purifiées, les exopolysaccharides modifiés et les Polyaminosaccharides alkylsulfonnés.

Comme agents cicatrisants préférés selon l'invention, on utilisera l'huile de tamanu, l'acexamate de sodium, des extraits de miel, des extraits de prêle, des extraits d'helichryse, et leurs mélanges.

### Agents astringents

Par 'agents astringents', on entend selon l'invention des agents permettant de lutter contre la dilatation des follicules sébacés.

Comme agents astringents utilisables dans la composition selon l'invention, on peut citer le Laricyl LS8865^{®} de Cognis, le Phytofirm LS9120^{®} de Cognis , le Tanlex VE/VB^{®} de Ichimaru Pharcos, la laponite, les sels d'aluminium, les extraits de centella (ex Plantactiv centella de Cognis), le Varisoft 432 CG^{®} de Degussa, les extraits de marron d'inde, les extraits de mauve, d'Hammamelis, l'Almondermin LS 3380^{®} de Cognis, les extraits de bardane, l'Extrapone 9 spécia^{®}l de Symrise, les extraits de scutellaria, les extrais d'Ulmaire (ex Cytobiol Ulmaire de Libiol), l'Herb extract B1348^{®} de Bell flavors & fragrances, les extraits d'acacia, d'orme, de saule blanc, de canelle, de bouleau, de reine des prés, les sapogénines de panama, le phenolsulfonate de zinc de Interchemical, des extraits de gentiane, de concombre, de noyer, le mélange Epilami de Alban Muller.

Comme agents astringents préférés selon l'invention, on utilisera les extrais de scutellaria, les extraits d'ulmaire, les extraits de reine des près, les extraits de gentiane, les extraits de bardane et leurs mélanges.

### Essais

Des essais ont été effectués au moyen d'un dispositif à LEDs GentleWaves™ de la société Light Bioscience émettant à une longueur d'onde dominante d'environ 595 nm (lumière jaune visible) ainsi qu'à une deuxième longueur d'onde de 870 nm, à une moindre puissance, avec une puissance surfacique totale de 5mW/cm², la lumière étant pulsée avec un cycle 250 ms d'émission et 100 ms entre les émissions. La puissance de la longueur d'onde à 870 nm représente au plus 10 à 15% de la puissance de la longueur d'onde dominante à 595nm.

### Essai 1

Essai mené sur 94 femmes à peau grasse : 3 groupes sont traités sur une moitié du visage uniquement de façon aléatoire avec l'invention :
- Groupe 1 : 35s deux fois par semaine pendant quatre semaines (8 séances) le sujet applique sa crème cosmétique habituelle sur tout le visage, matin et soir.
- Groupe 2: 35s deux fois par semaine pendant quatre semaines suivi de 35s une fois par semaine pendant quatre semaines (douze séances), le sujet applique sa crème cosmétique habituelle sur tout le visage matin et soir
- Groupe 3: 35s deux fois par semaine pendant quatre semaines suivi de 35s une fois par semaine pendant quatre semaines (douze séances), avec application des compositions A et B , comparé à l'utilisation de la crème habituelle seule sur l'autre moitié de visage non traitée par l'invention.

### Résultats du Sebutape^{®}

Les valeurs de sébutape après 2, 4 ou 8 semaines de traitement ont été comparées aux valeurs de sébutape avant traitement.

Dans le groupe 1, la moyenne des différences est statistiquement significative et égale à -0.3062 dès 2 semaines de traitement par l'invention alors que cette différence n'est pas statistiquement significative du côté non traité par l'invention.

Dans le groupe 3, la moyenne des différences est statistiquement significative et égale à -0.373 dès 4 semaines et cette différence est maintenue de façon statistiquement significative à 8 semaines du côté traité par l'invention et les compositions A et B.

Résultats du Sebumètre : Les valeurs de sébumètre après 2, 4 ou 8 semaines de traitement ont été comparées aux valeurs de sébumètre avant traitement.

Dans le groupe 2, la moyenne des différences est statistiquement significative et égale à -19.7813 dès 2 semaines de traitement. Cette différence est maintenue (= - 16.6563) statistiquement significative à 4 semaines de traitement par l'invention alors que cette différence n'est pas statistiquement significative du côté non traité.

Dans le groupe 3, la moyenne des différences est statistiquement significative et égale à -24.800 dès 4 semaines de traitement. Cette différence est maintenue (= -23.967) statistiquement significative à 8 semaines de traitement par l'invention plus les compositions A et B alors que cette différence n'est pas statistiquement significative du côté non traité par l'invention.

Résultats du Dermascore : Les valeurs du dermascore après 2, 4 ou 8 semaines de traitement ont été comparées aux valeurs du dermascore avant traitement par l'invention.

Dans le groupe 2, la moyenne des différences est statistiquement significative et égale à -0.1438 dès 2 semaines de traitement. Cette différence n'est pas statistiquement significative du côté non traité par l'invention.

Dans le groupe 3, la moyenne des différences est statistiquement significative et égale à -0.227 dès 4 semaines de traitement par l'invention plus les compositions A et B alors que cette différence n'est pas statistiquement significative du côté non traité à 4 semaines de traitement par l'invention sans les compositions A et B.

L'efficacité du traitement a été très bien perçue par les volontaires. La peau paraît moins grasse (97% G3 dès 2 semaines), plus lisse (97% G1 et G3 à 8 semaines), plus saine (97% G3 à 8 semaines), le grain de peau paraît plus fin (97% G3 à 4 semaines), les pores plus resserrés (93% G3), moins visibles (93% G3 dès 2 semaines).

La comparaison des trois modalités de traitement sur le pourcentage de satisfaction des femmes sur le critère « visibilité des pores » à 2 et à 4 semaines montre une différence significative en faveur du groupe de femmes utilisatrices du traitement avec la routine de soin comportant les compositions A et B (groupe 3) par rapport à celles qui ont la même modalité d'utilisation du dispositif mais sans association avec les compositions A et B (groupe 2).

Toutes les modalités de traitement précisées ci-dessus ont permis de diminuer de façon significative les excrétions sébacées mesurées au moyen du sébutape™ dès 4 semaines en comparaison du côté non traité. L'association du traitement par le dispositif avec un produit spécifique permet d'atteindre une diminution significative de ce même paramètre après 2 semaines.

### Essai 2

Essai mené sur 100 (60/40) femmes et hommes traités sur l'ensemble du visage de façon aléatoire par l'invention suivant le groupe : un groupe A de sujets est traité une fois 35s, deux fois par semaine, pendant quatre semaines. Un autre groupe B de sujets est traité deux fois consécutives 35s, deux fois par semaine, pendant quatre semaines. Un autre groupe C de sujets est traité une fois 35s, deux fois par jour matin et soir, deux fois par semaine, pendant quatre semaines. Un autre groupe D de sujets est traité une fois 35s, chaque jour, cinq fois par semaine du lundi au vendredi, pendant quatre semaines. Un autre groupe E de sujets est traité deux fois consécutives 35s, chaque jour, cinq fois par semaine du lundi au vendredi, pendant quatre semaines.

### Résultats sur la taille des pores :

L'étude sur la taille des pores a été faite par analyse et évaluation sur la base de photographies des sujets faites à la fin du traitement et comparées aux photographies faites avant le traitement par l'invention. Il a été démontré une différence significative dans l'apparence des pores pour les sujets traités dans le groupe D (7.6%), le groupe C (6.5%) et dans le groupe B (6.5%). Aucune différence n'a été mise en évidence sur l'apparence des pores dans les groupes A et E.

### Essai 3

Essai mené sur 124 sujets à peau grasse traités par l'invention sur la moitié du visage (gauche ou droite) de façon aléatoire pendant 70 secondes, 1 fois par jour 5 jours de suite pendant 4 semaines.

On observe une diminution statistiquement significative (p< 0.001) de la taille des pores sur la moitié du visage traitée pendant 1 mois par l'invention par rapport à l'état initial avant traitement.

On observe à 1 mois de traitement une différence significative égale à 0.16 (p<0.05) des variations d'excrétion du sébum par rapport à l'état de base entre le côté traité par l'invention et le côté non traité par l'invention

Une évaluation effectuée par un expert en aveugle par rapport à la moitié du visage traité ou non traité pendant 1 mois par l'invention. a montré les résultats suivants : sur la moitié du visage traité pendant 1 mois par l'invention l'expert a évalué une peau moins grasse et moins brillante chez 61% et 62% des sujets respectivement alors que du côté non traité par l'invention à 1 mois l'expert a évalué une peau moins grasse et moins brillante chez 38 et 42 % des sujets respectivement. Ces différences sont statistiquement significatives. L'effet du traitement par le sujet a été évalué par des auto-questionnaires après 1 mois de traitement par l'invention : les sujets ont considéré que leur peau était moins grasse sur la moitié du visage traitée (66%) et 50% pour la moitié du visage non traitée par l'invention. Au niveau de la taille des pores, les sujets ont considéré que les pores étaient moins visibles (63%) et resserrés (66%) sur la moitié du visage traitée et 48% et 47% respectivement pour la moitié du visage non traitée.

L'invention sera mieux comprise à la lecture de la description détaillée qui va suivre, d'exemples de réalisation de l'invention et à l'examen du dessin annexé sur lequel :
- les figures 1 à 3 illustrent des spectres de lumière (Intensité I en fonction de la longueur d'onde (λ) émise conformément à l'invention, et
- la figure 4 illustre une succession de pulses conforme à un exemple de mise en oeuvre de l'invention.

### Dispositif

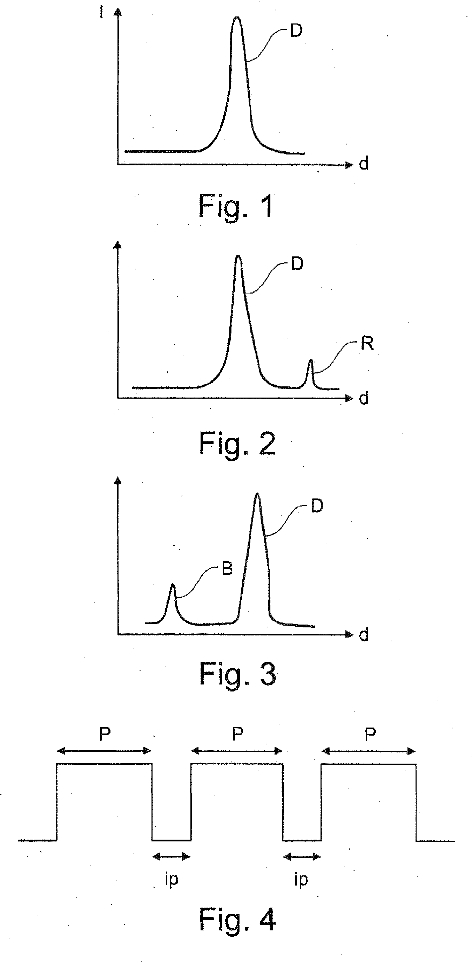

Le procédé selon l'invention est par exemple mis en oeuvre au moyen d'un dispositif d'application de lumière sur la peau, comportant au moins une source de lumière quasi-mononochromatique d'origine artificielle permettant d'émettre la première longueur d'onde D, comme illustré sur la figure 1, et une ou plusieurs fenêtre(s) de sortie de la lumière émise.

Le dispositif peut en outre être configuré pour émettre une deuxième lumière quasi-mononochromatique d'origine artificielle ayant un pic R de longueur d'onde comprise entre 700 et 1000 nm, mieux entre 800 et 900 nm, par exemple de l'ordre d'environ 870 nm, comme illustré à la figure 2. Cette lumière est une lumière rouge ou infrarouge. La source de la lumière rouge ou infrarouge peut être la même que ou différente de celle à l'origine de la première lumière mentionnée ci-dessus. Le dispositif peut comporter une unique source de lumière configurée pour émettre deux lumières quasi-monochromatiques différentes. En variante, le dispositif peut comporter deux sources différentes émettant chacune au moins une lumière quasi-monochromatique différente, les deux sources pouvant être activées simultanément ou successivement.

Le dispositif peut encore être configuré pour émettre une troisième lumière quasi-monochromatique d'origine artificielle, additionnelle, ayant un pic B de longueur d'onde compris par exemple entre 400 et 450 nm, notamment de l'ordre de 410 à 420 nm, comme illustré sur la figure 3. Cette lumière est une lumière bleue. Le dispositif peut comporter une source de lumière additionnelle différente de la ou des sources de lumière émettant la première lumière D et la lumière rouge ou infrarouge R.

Le dispositif peut être configuré pour émettre une lumière pulsée avec des pulses d'une durée p comprise entre 100 et 500 ms, mieux entre 200 et 300 ms, par exemple de l'ordre de 250 ms, et des interpulses *ip* d'une durée comprise entre 50 et 200 ms, mieux entre 70 et 150 ms, par exemple de l'ordre de 100 ms, comme illustré sur la figure 4. En variante, le dispositif peut être configuré pour émettre une lumière continue.

La puissance surfacique de la lumière émise peut être inférieure à 40 mW/cm², de préférence comprise entre 1 et 20 mW/cm², mieux comprise entre 1 et 10 mW/cm².

L'énergie surfacique émise peut être inférieure à 4 J/cm², mieux comprise entre 20 mJ/cm² et 1 J/cm², par exemple de l'ordre de 175 mJ/cm².

Le dispositif peut être configuré pour émettre de la lumière pendant une certaine durée de fonctionnement prédéfinie, puis s'éteindre automatiquement. La durée de fonctionnement peut être inférieure à 20 minutes, par exemple comprise entre 20 et 100 secondes, par exemple être de l'ordre de 35 secondes ou de 70 secondes dans certains exemples de réalisation.

Le dispositif peut être configuré pour que la puissance du pic dominant de la lumière R soit au moins inférieur au quart de la puissance du pic dominant de la lumière D.

Le dispositif peut comporter des moyens pour conduire la lumière émise par la source à proximité de la peau, par exemple une ou plusieurs fibres optiques. Le dispositif peut comporter un faisceau de fibres optiques conduisant la lumière émise par la source vers une pluralité de zones de la peau à traiter.

Le dispositif peut être configuré pour permettre de traiter l'ensemble de la peau à traiter du sujet de manière statique. Il peut par exemple prendre la forme d'un casque destiné à venir recouvrir la tête, pour permettre le traitement du cuir chevelu gras. Il peut en variante comporter plusieurs panneaux, chaque panneau portant une ou plusieurs sources de lumière, destinés à être disposés devant le visage et sur les côtés du visage d'un sujet.

Le dispositif peut comporter des reliefs destiné à être placés en contact avec la peau du sujet lorsque le dispositif est placé sur le sujet pour effectuer le traitement, ces reliefs permettant de garantir une distance adéquate entre la peau à traiter et la ou les sources de lumière.

En variante, le dispositif comprend deux panneaux de LEDs incurvés qui pivotent l'un par rapport à l'autre, un bras à hauteur ajustable, une unité de contrôle et un système d'alimentation. Chaque panneau peut être constitué d'une matrice d'au moins 1000 LEDs.

En variante, le dispositif comprend un seul panneau de LEDs incurvé relié à une unité de contrôle et à un système d'alimentation. Chaque panneau peut être constitué d'une matrice d'au moins 800 LEDs.

L'invention n'est pas limitée aux exemples de réalisation qui viennent d'être décrits.

L'expression « comportant un » doit se comprendre comme étant synonyme de « comportant au moins un ».

## Revendications

1. Procédé de traitement cosmétique non-thérapeutique de la peau grasse non acnéique, la peau grasse non acnéique étant la peau du visage, comportant l'étape consistant à :
- exposer ladite peau grasse non acnéique à une première lumière quasi-monochromatique d'origine artificielle ayant un pic dominant de longueur d'onde comprise entre 400 et 650 nm,
dans lequel on expose également, notamment simultanément ou non, la peau grasse non acnéique à une deuxième lumière quasi-monochromatique d'origine artificielle ayant un pic dominant de longueur d'onde comprise entre 800 et 900 nm.

2. Procédé selon la revendication précédente, dans lequel la deuxième lumière quasi-monochromatique d'origine artificielle a un pic dominant de longueur d'onde de l'ordre de 870 nm.

3. Procédé selon l'une des revendications précédentes, dans lequel ladite lumière, notamment la première lumière, est pulsée avec des pulses d'une durée comprise entre 100 et 500 ms.

4. Procédé selon l'une quelconque des revendications précédentes, dans lequel la lumière, notamment la première lumière, est pulsée avec des interpulses d'une durée comprise entre 50 et 200 ms.

5. Procédé selon l'une quelconque des revendications précédentes, dans lequel la lumière reçue par la peau grasse non acnéique est de puissance surfacique inférieure ou égale à 40 mW/cm².

6. Procédé selon l'une quelconque des revendications précédentes, dans lequel on expose la peau grasse non acnéique à la lumière, notamment la première lumière, pendant une durée inférieure à 20 minutes.

7. Procédé selon l'une quelconque des revendications précédentes, dans lequel on effectue ledit traitement au moins une fois dans la journée et au moins un jour par semaine.

8. Procédé selon l'une quelconque des revendications précédentes, dans lequel on effectue ledit traitement au moins une fois par semaine et pendant une durée comprise entre deux et douze semaines.

9. Procédé selon l'une quelconque des revendications précédentes, dans lequel on expose la peau grasse non acnéique à une troisième lumière quasi-monochromatique, bleue, ayant de préférence un pic dominant de longueur d'onde comprise entre 400 et 450 nm.

10. Procédé selon l'une quelconque des revendications précédentes, dans lequel la ou les source(s) de lumière comporte(nt) l'un au moins d'une LED, d'une matrice de LEDs, d'une OLED, d'un laser, d'une lampe à incandescence munie d'un filtre dichroïque.

11. Procédé selon l'une quelconque des revendications précédentes, dans lequel la ou les source(s) de lumière comporte(nt) au moins une LED quasi-monochromatique.

12. Procédé selon l'une quelconque des revendications précédentes, comportant l'étape consistant à :
- appliquer sur la peau grasse non acnéique, préalablement à l'exposition à ladite lumière, un produit cosmétique.

13. Procédé selon l'une quelconque des revendications précédentes, comportant l'étape consistant à :
- appliquer sur la peau grasse non acnéique, après l'exposition à ladite lumière, un produit cosmétique.

14. Procédé selon l'une quelconque des revendications précédentes, comprenant l'application topique sur la peau grasse non acnéique d'une composition comprenant de l'acide n-octanoyl-5-salicylique.

## Patentansprüche

1. Kosmetisches nicht-therapeutisches Verfahren zur Behandlung fettiger Haut ohne Akne, wobei die fettige Haut ohne Akne die Haut des Gesichtes ist, umfassend die folgenden Schritte:
- Exponieren der fettigen Haut ohne Akne gegenüber einem ersten quasi-monochromatischen Licht künstlichen Ursprungs mit einem Wellenlängen-Hauptpeak von 400 bis 650 nm,
wobei, insbesondere gleichzeitig oder nicht gleichzeitig, die fettige Haut ohne Akne auch einer zweiten quasi-monochromatischen Lichtquelle künstlichen Ursprungs mit einem Wellenlängen-Hauptpeak von 800 bis 900 nm ausgesetzt wird.

2. Verfahren nach dem vorangehenden Anspruch, wobei das zweite quasi-monochromatische Licht künstlichen Ursprungs einen Wellenlängen-Hauptpeak in der Größenordnung von 870 nm aufweist.

3. Verfahren nach einem der vorangehenden Ansprüche, wobei das Licht, insbesondere das erste Licht, mit Pulsen einer Dauer von 100 und 500 ms gepulst wird.

4. Verfahren nach einem der vorangehenden Ansprüche, wobei das Licht, insbesondere das erste Licht, mit Zwischenpulsen einer Dauer von 50 und 200 ms gepulst wird.

5. Verfahren nach einem der vorangehenden Ansprüche, wobei das von der fettigen Haut ohne Akne aufgenommene Licht eine Oberflächenleistungsdichte von kleiner oder gleich 40 mW/cm² aufweist.

6. Verfahren nach einem der vorangehenden Ansprüche, wobei die fettige Haut ohne Akne Licht, insbesondere dem ersten Licht, während einer Dauer unter 20 Minuten ausgesetzt wird.

7. Verfahren nach einem der vorangehenden Ansprüche, wobei die Behandlung mindestens einmal pro Tag und mindestens einen Tag pro Woche durchgeführt wird.

8. Verfahren nach einem der vorangehenden Ansprüche, wobei die Behandlung mindestens einmal pro Woche und während einer Dauer zwischen 2 und 12 Wochen durchgeführt wird.

9. Verfahren nach einem der vorangehenden Ansprüche, wobei die fettige Haut ohne Akne einem dritten quasi-monochromatischen blauen Licht mit vorzugsweise einem Wellenlängen-Hauptpeak von 400 und 450 nm ausgesetzt wird.

10. Verfahren nach einem der vorangehenden Ansprüche, wobei die Lichtquelle oder die Lichtquellen eine von mindestens einer LED, einer Matrix von LEDs oder einer OLED, einem Laser, einer Inkandeszenzlampe, die mit einem dichroischen Filter versehen ist, umfasst.

11. Verfahren nach einem der vorangehenden Ansprüche, wobei die Lichtquelle oder die Lichtquellen mindestens eine quasi-monochromatische LED umfasst.

12. Verfahren nach einem der vorangehenden Ansprüche, umfassend den folgenden Schritt:
- Aufbringen eines kosmetischen Produkts auf die fettige Haut ohne Akne vor der Exposition gegenüber dem Licht.

13. Verfahren nach einem der vorangehenden Ansprüche, umfassend den folgenden Schritt:
- Aufbringen eines kosmetischen Produkts auf die fettige Haut ohne Akne nach der Exposition gegenüber dem Licht.

14. Verfahren nach einem der vorangehenden Ansprüche, das die topische Anwendung auf die fettige Haut ohne Akne einer Zusammensetzung umfasst, die n-Octanoyl-5-salicylsäure umfasst.

## Claims

1. A non-therapeutic cosmetic treatment method for non-acneic oily skin, the non-acneic oily skin being the skin of the face, including the step consisting of:
- exposing said non-acneic oily skin to a first quasi-monochromatic light of artificial origin having a dominant peak with a wavelength comprised between 400 and 650 nm,
wherein the non-acneic oily skin is also exposed, notably simultaneously or not to a second quasi-monochromatic light of artificial origin having a dominant peak with a wavelength comprised between 800 and 900 nm.

2. The method according to the preceding claim, wherein the second quasi-monochromatic light of artificial origin has a dominant peak with a wavelength of the order of 870 nm.

3. The method according to one of the preceding claims, wherein said light, notably the first light, is pulsed with pulses with a period comprised between 100 and 500 ms.

4. The method according to any of the preceding claims, wherein said light, notably the first light, is pulsed with interpulses with a period comprised between 50 and 200 ms.

5. The method according to any of the preceding claims, wherein the light received by the non-acneic oily skin is of a surface power of less than or equal to 40 mW/cm².

6. The method according to any of the preceding claims, wherein the non-acneic oily skin is exposed to light, notably the first light, for a period of less than 20 minutes.

7. The method according to any of the preceding claims, wherein said treatment is carried out at least once during the day and for a least one day per week.

8. The method according to any of the preceding claims, wherein said treatment is carried out at least once a week and for a period comprised between two and twelve weeks.

9. The method according to any of the preceding claims, wherein the non-acneic oily skin is exposed to a third blue quasi-monochromatic light preferably having a dominant peak comprised between 400 and 450 nm.

10. The method according to any of the preceding claims, wherein the light source(s) include(s) at least one LED, one LED array, one OLED, one laser, one incandescent lamp provided with a dichroic filter.

11. The method according to any of the preceding claims, wherein the light source(s) include(s) at least one quasi-monochromatic LED.

12. The method according to any of the preceding claims, including the step consisting of:
- applying on the non-acneic oily skin, prior to exposing it to said light, a cosmetic product.

13. The method according to any of the preceding claims, including the step consisting of:
- applying on the non-acneic oily skin, after exposing it to said light, a cosmetic product.

14. The method according to any of the preceding claims, comprising the topical application on the non-acneic oily skin of a composition comprising n-octanoyl-5-salicylic acid.
